# EUROPÄISCHE PATENTSCHRIFT

(11) **EP 0 560 178 B1**
(45) Veröffentlichungstag und Bekanntmachung des Hinweises auf die Patenterteilung: **20.11.1996**
(21) Anmeldenummer: 93103302.1
(22) Anmeldetag: 02.03.1993
(51) Int. Cl.: C07D 239/42, C07D 239/46, C07D 239/52, C07D 239/56, C07D 239/58, C07D 251/42, C07D 251/46

(54) **Verfahren zur Herstellung von Sulfonylharnstoffen**
Process for the preparation of sulfonylureas
Procédé pour la préparation de dérivés de sulfonylurées

(30) Priorität: 07.03.1992 DE 4207242
(43) Veröffentlichungstag der Anmeldung: 15.09.1993
(73) Patentinhaber: Hoechst Schering AgrEvo GmbH, 13342 Berlin (DE)
(72) Erfinder: Schlegel, Günter, Dr., W-6237 Liederbach (DE)

(56) Entgegenhaltungen:
- EP-A- 0 004 163
- EP-A- 0 131 258
- EP-A- 0 342 569
- DE-A- 2 257 240

## Beschreibung

Die Erfindung betrifft Verfahren zur Herstellung von heterocyclisch substituierten Sulfonylharnstoffherbiziden, speziell der Verbindungen der Formel I, 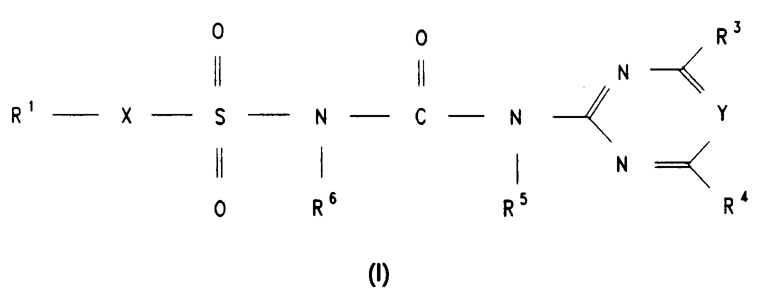
worin
- X: Sauerstoff, -O-NR²- oder -SO₂-NR²-,
- Y: Stickstoff oder CH,
- R¹: (C₁-C₆)Alkyl, (C₂-C₆)Alkenyl oder (C₂-C₆)Alkinyl, wobei jeder der letztgenannten drei Reste unabhängig voneinander unsubstituiert oder durch einen oder mehrere Reste aus der Gruppe Halogen, (C₁-C₄)Alkoxy und [(C₁-C₄)Alkoxy]-carbonyl substituiert ist,
oder, im Falle von X = Sauerstoff, auch Phenyl, das unsubstituiert oder durch einen oder mehrere Reste aus der Gruppe Halogen, Nitro, (C₁-C₄)Alkyl, (C₁-C₄)Haloalkyl, (C₁-C₄)Alkoxy, (C₁-C₄)Haloalkoxy und [(C₁-C₄)Alkoxy]-carbonyl substituiert ist,
- R²: Wasserstoff, (C₁-C₆)Alkyl, (C₂-C₆)Alkenyl, (C₂-C₆)Alkinyl oder (C₃-C₆)Cycloalkyl,
- R³, R⁴: unabhängig voneinander Wasserstoff, (C₁-C₄)Alkyl oder (C₁-C₄)Alkoxy, wobei jeder der letztgenannten beiden Reste unsubstituiert oder durch einen oder mehrere Reste aus der Gruppe Halogen, Alkoxy und Alkylthio substituiert ist, oder Halogen, (C₁-C₄)Alkylthio, (C₁-C₄)Alkylamino oder Di[(C₁-C₄)alkyl]-amino und
- R⁵: Wasserstoff oder (C₁-C₄)Alkyl und
- R⁶: Wasserstoff bedeuten
sowie deren Salze mit Säuren oder Basen.

Formel I umfaßt auch alle nicht genannten, durch ihre spezifische Raumform definierbaren möglichen Stereoisomeren, wie Enantiomere, Diastereomere, Z- und E-Isomere, welche die in Formel I genannte Verknüpfung von Atomen aufweisen. Solche Verbindungen der Formel I enthalten beispielsweise ein oder mehrere asymmetrische C-Atome oder auch Doppelbindungen, die in der allgemeinen Formel I nicht gesondert angegeben sind. Die Stereoisomere können nach üblichen Methoden aus Gemischen der Stereoisomeren enthalten werden oder auch durch stereoselektive Reaktionen in Kombination mit dem Einsatz von stereochemisch reinen Ausgangsstoffen hergestellt werden.

Die Verbindungen der Formel I können Salze bilden, bei denen der Wasserstoff der -SO₂-NH-Gruppe durch ein, insbesondere für die Landwirtschaft geeignetes Kation ersetzt wird. Diese Salze sind beispielsweise Metall-, insbesondere Alkalisalze (z. B. mit Na⁺ oder K⁺ als Kation) oder Erdalkalisalze, oder auch Ammoniumsalze oder Salze mit organischen Aminen. Ebenso kann Salzbildung durch Anlagerung einer starken Säure an den Pyrimidinteil der Verbindung der Formel I erfolgen. Geeignete Säuren hierfür sind starke anorganische und organische Säuren, beispielsweise HCl, HBr, H₂SO₄ oder HNO₃.

Verbindungen der Formel I sind bekannt und werden als Pflanzenschutzmittel mit herbizider Wirkung eingesetzt; siehe EP-A-013258 (US-A-4,601,747), EP-A-0342569 (US-A-5,104,443) und EP-A-4163 (US-A-4,191,553). In den genannten Druckschriften sind auch einige Verfahren zitiert oder beschrieben, nach denen Verbindungen der Formel I hergestellt werden können.

Nachteilig bei den bekannten Verfahren ist die Verwendung von Chlorsulfonylisocyanat (CSI), dessen hohe Reaktivität sicherheitstechnische Probleme und dessen schlechte Zugänglichkeit hohe Kosten verursachen. Die bekannten Verfahren sind daher sowohl aus sicherheitstechnischer als auch ökonomischer Sicht für die Durchführung im industriellen, großtechnischen Maßstab ungünstig.

Es wurde nun ein neues Verfahren gefunden, nach dem Verbindungen der Formel I durch Umsetzung leicht zugänglicher Ausgangsmaterialien überraschend effektiv hergestellt werden können.

Gegenstand der vorliegenden Erfindung ist ein Verfahren zur Herstellung der genannten Verbindungen der Formel I oder deren Salze, dadurch gekennzeichnet, daß man Verbindungen der Formel II,

R¹ - X - R⁷ (II)

worin R¹ und X wie in Formel I definiert sind und R⁷ Wasserstoff, ein quarternäres Ammoniumion oder ein Äquivalent eines ein-, zwei- oder mehrwertigen Metallkations ist,
mit Verbindungen der Formeln III, IV und V,

R⁸ - OCN (III)

SO₂Cl₂ (IV)

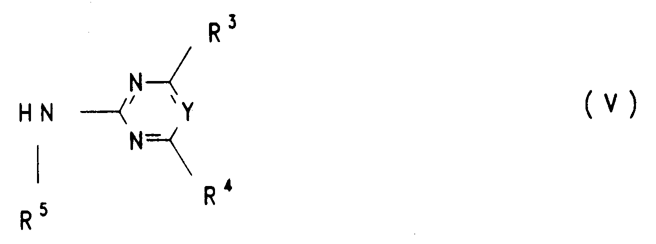

in denen R³, R⁴, R⁵, X und Y wie in Formel I definiert sind und R⁸ Wasserstoff, ein quarternäres Ammoniumion oder ein Äquivalent eines ein-, zwei- oder mehrwertigen Metallkations bedeutet, umsetzt.

Unter den erfindungsgemäßen Verfahren zur Herstellung der Verbindungen der Formel I sind diejenigen von besonderem Interesse, in denen R¹X N-[(C₁-C₆)-Alkylsulfonyl]-N-[(C₁-C₃)-alkyl]-amino oder [(C₁-C₄)Alkoxy]-phenoxy, R³ und R⁴ unabhängig voneinander (C₁-C₂)Alkyl oder (C₁-C₂)Alkoxy und R⁵ Wasserstoff oder Methyl und R⁶ Wasserstoff bedeuten.

Vorzugsweise ist R¹X N-[(C₁-C₃)-Alkylsulfonyl]-N-[(C₁-C₂)-alkyl]-amino, insbesondere N-(Methylsulfonyl)-N-(methyl)-amino, N-(Methylsulfonyl)-N-(ethyl)-amino, N-(Ethylsulfonyl)-N-(methyl)-amino oder N-(n-Propylsulfonyl)-N-(methyl)-amino; vorzugsweise ist R¹X auch (C₁-C₃)-Alkoxyphenoxy, insbesondere 2-Methoxyphenoxy, 2-Ethoxyphenoxy, 2-n-Propoxy-phenoxy oder 2-Isopropoxyphenoxy.

Vorzugsweise sind R³ und R⁴ unabhängig voneinander (C₁-C₂)Alkyl oder (C₁-C₂)Alkoxy, insbesondere Methyl oder Methoxy.

Beispiele für R⁷ and R⁸ sind Alkali- oder Erdalkalimetallkationen wie Natrium-, Kalium-, Magnesium- und Calzium-ionen. Für Metallkationen mit einer Wertigkeit von mehr als 1 sind entsprechend zwei oder mehrere Reste der Formel R¹-X in Verbindungen der Formel II sowie mehrere Reste OCN in Verbindungen der Formel III mit den Metallionen verbunden. Beispiele für R⁷ and R⁸ sind auch quarternäre Ammoniumionen wie Tetraalkylammonium, Trialkylarylammonium, Dialkyldiarylammonium, Alkyltriarylammonium und Tetraarylammonium, wobei die Alkylreste gegebenenfalls substituiert sein können, z. B. durch Alkoxy oder Aryl.

In den genannten Formeln und im folgenden können kohlenwasserstoffhaltige Reste wie z.B. Alkyl-, Alkoxy-, Haloalkoxy- und Alkylthioreste sowie die entsprechenden ungesättigten und/oder substituierten Reste im Kohlenwasserstoffteil jeweils geradkettig oder verzweigt sein. Alkylreste, auch in den zusammengesetzten Bedeutungen wie Alkoxy, Haloalkyl usw., bedeuten Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet, Fluor, Chlor, Brom oder Jod. Haloalkyl bedeutet Alkyl, das durch ein oder mehrere Atome aus der Gruppe Halogen substituiert ist; Haloalkyl ist beispielsweise CF₃, CHF₂, CH₂CF₃. Aryl ist beispielsweise Phenyl, Naphthyl, Tetrahydronaphthyl, Indanyl, Fluorenyl und ähnliches, vorzugsweise Phenyl. Substituiertes Aryl oder substituiertes Phenyl bedeutet vorzugsweise Aryl bzw. Phenyl, das durch einen oder mehrere, vorzugsweise 1 bis 3 Reste aus der Gruppe Halogen, Alkyl, Haloalkyl, Haloalkoxy, Nitro, Cyano, Alkoxycarbonyl, Alkanoyl, Carbamoyl, Mono- und Di-alkylaminocarbonyl, Mono- und Dialkylamino, Alkylsulfinyl oder Alkylsulfonyl substituiert ist, wobei bei den alkylhaltigen Resten solche mit 1 bis 4 C-Atomen, insbesondere 1 bis 2 C-Atomen bevorzugt sind; besonders bevorzugt sind dabei Methyl, Methoxy und Chlor.

Die Ausbeuten der entstehenden Sulfonylharnstoffe der Formel I sind nach dem erfindungsgemäßen Verfahren vergleichsweise hoch, z. B. bei 80 % und mehr, wobei Reinheiten von mehr als 94 Gew.-% meist schon ohne aufwendige Reinigungsschritte erhalten werden.

Die Durchführung der Reaktion verläuft vorzugsweise in zwei oder mehreren Schritten. Zunächst werden dabei die Verbindungen der Formeln II, III und IV miteinander umgesetzt. Danach wird die so erhaltene Reaktionsmischung mit Verbindungen der Formel V versetzt. Bei der Zusammengabe der Reaktanden auftretende Zwischenstufen können in der Regel isoliert werden. Das Gesamtverfahren ist aber auch als Eintopfverfahren durchzuführen.

Die Reaktionstemperaturen für die Umsetzungen der Komponenten II, III und IV liegen vorzugsweise zwischen 0 °C und +200 °C, insbesondere zwischen +8 °C und + 135 °C, ganz besonders zwischen +20 °C und +90 °C.

Die Reaktionstemperatur für die Umsetzung der Reaktionsmischung aus II, III und IV mit dem Aminoheterocyclus V liegt vorzugsweise zwischen -20 °C und + 120 °C, insbesondere zwischen -5 °C und +80 °C.

Das erfindungsgemäße Verfahren kann ohne Lösungsmittel durchgeführt werden. Oft ist es jedoch vorteilhaft, das Verfahren oder einzelne Verfahrensschritte in Gegenwart von unter den Reaktionsbedingungen inerten anorganischen oder organischen Lösungsmitteln oder in Gemischen dieser Lösungsmittel durchzuführen. Es kann auch vorteilhaft sein, zwischen den Teilschritten des Verfahrens das Lösungsmittel zu wechseln.

Beispiele für geeignete organische Lösungsmittel sind aprotische polare organische Lösungsmittel, wie aliphatische oder aromatische Nitrile, N,N-Dialkyl-alkanoylamide, Dialkylsulfoxide, Polyalkylenglykoldialkylether und N-alkylierte cyclische Amide, und aliphatische oder vorzugsweise aromatische, gegebenenfalls halogenierte Kohlenwasserstoffe oder Gemische der genannten organischen Lösungsmittel. Beispiele für geeignete anorganische Lösungsmittel sind flüssiges Schwefeldioxid und flüssige Blausäure sowie deren Mischungen. Auch Mischungen der genannten organischen und anorganischen Lösungsmitteln sind möglich.

Bevorzugt sind Lösungsmittel wie z. B. Acetonitril, Propionitril, Benzonitril, Dimethylformamid, Dimethylsulfoxid, Sulfolan, N-Methylpyrrolidon, Ethylenglykoldialkylether, Di-, Tri- oder Tetraethylenglykoldialkylether, insbesondere -dimethyl oder - diethylether, Toluol, Xylol, Chlorbenzol, flüssiges Schwefeldioxid oder auch Mischungen aus zwei oder mehreren der genannten Lösungsmittel. Es kann auch speziell vorteilhaft sein, den ersten Teilschritt des Verfahrens, d. h. Umsetzungen von II, III und IV oder von II und IV, beispielsweise in einem polaren aprotischen organischen oder anorganischen Lösungsmittel, wie Acetonitril oder flüssiges Schwefeldioxid, und den folgenden oder die folgenden Schritte beispielsweise in weniger polaren aprotischen organischen Lösungsmittel, wie Toluol oder Xylol, durchzuführen.

Für den Fall, daß die Verbindungen der Formel II und/oder der Formel III in dem gewählten Lösungsmittel nicht vollständig löslich sind, kann die Reaktion durch sehr intensive Durchmischung der Reaktionspartner beispielsweise mittels starkem Rühren oder durch Ultraschall beschleunigt werden.

Zur Umsetzung der Verbindungen der Formeln II, III und IV kann man beispielsweise zunächst die Verbindungen der Formeln III und IV umsetzen, beispielsweise bei einer Reaktionstemperatur von 0 bis 80°C ohne Lösungsmittel oder in Gegenwart eines der genannten aprotischen polaren organischen oder aprotischen polaren anorganischen Lösungsmittel, wobei eine Additionsverbindung nicht geklärter Struktur (Addukt 1) entsteht. Danach wird die Umsetzung von II mit dem vorher erhaltenen Addukt 1, am einfachsten in Form der vorher erhaltenen Reaktionsmischung, gegebenenfalls unter Erwärmen durchführt, wobei wiederum eine Additionsverbindung nicht geklärter Struktur (Addukt 2) entsteht, die für die erfindungsgemäße Umsetzung mit der Verbindung der Formel V geeignet ist. Die Umsetzung mit der Verbindung II erfolgt beispielsweise bei einer Reaktionstemperatur von 0 bis 200°C, vorzugsweise von +8 °C bis + 135 °C, insbesondere von +20 °C bis +90 °C in Gegenwart eines der genannten aprotischen polaren organischen oder aprotischen polaren anorganischen Lösungsmittel. Es ist häufig von Vorteil, die genannten Umsetzungen der Verbindungen II, III und IV anstatt bei konstanter Temperatur unter Anwendung eines ansteigenden Temperaturgradienten vorzunehmen.

Nach Ergebnissen von Untersuchungen mit Nachweisreaktionen und spektroskopischen Methoden bestehen die intermediär entstehenden Additionsverbindungen nicht wie zunächst vermutet aus einem Isocyanat der Formel R¹XSO₂-N=C=O, obwohl zumindest die Umsetzung von Addukt 2 mit dem Amin der Formel V zum substituierten Harnstoff der Formel I formal gesehen dasgleiche ergibt, das die Umsetzung eines Isocyanats der Formel R¹XSO₂NCO mit dem Amin der Formel V erwarten läßt.

Gegenstand der Erfindung sind deshalb auch die genannten Addukte 1 und 2, die durch die genannten Varianten der Umsetzungen von Verbindungen III und IV bzw. der darauffolgenden Umsetzung mit Verbindung II gemäß dem erfindungsgemäßen Verfahren erhältlich sind.

In einer anderen Verfahrensweise kann man die Verbindungen der Formeln II, III und IV auch gemeinsam vorlegen, vorzugsweise bei niedriger Temperatur bis -10°C oder weniger, und gemeinsam auf Reaktionstemperatur erwärmen. In einer weiteren Verfahrensvariante kann man auch die Verbindungen der Formeln II und III gemeinsam vorlegen und das Sulfurylchlorid (Verbindung der Formel IV) vor Erwärmen auf die Reaktionstemperatur oder bei der Reaktonstemperatur für die Umsetzung der Verbindung der Formel II zugeben.

Die Umsetzung der Verbindungen der Formeln II, III und IV wird vorzugsweise unter aprotischen Bedingungen durchgeführt.

Im Falle, daß R⁷ oder R⁸ oder beide jeweils Wasserstoff bedeuten, ist es in der Regel zweckmäßig, für jedes Moläquivalent Wasserstoff 1 Moläquivalent einer Hilfsbase einzusetzen. Als Hilfsbase können anorganische Basen wie beispielsweise Erdalkalimetallcarbonate und/oder -hydrogencarbonate, Alkalimetallcarbonate und/oder -hydrogencarbonate und ähnliche Basen oder organische Basen wie beispielsweise Trialkylamine verwendet werden.

Es ist in der Regel vorteilhaft, im Falle R⁷ = Wasserstoff die Verbindungen der Formeln II und III, worin R⁸ ungleich Wasserstoff ist, im Molverhältnis II:III von höchstens etwa 1:2, insbesondere im Molverhältnis von etwa 1:2, einzusetzen, wobei mindestens ein Moläquivalent der Verbindung III als Hilfsbase dient. Verwendet man auch im Falle R⁷ = Wasserstoff ein Molverhältnis II:III von etwa 1:1, ist es für eine vollständige Umsetzung vorteilhaft, mindestens etwa ein Äquivalent einer anderen Hilfsbase bzw., wenn zusätzlich R⁸ = Wasserstoff ist, mindestens zwei Äquivalente einer anderen Hilfsbase einzusetzen. Als Hilfsbasen können die im vorigen Absatz erwähnten Basen eingesetzt werden.

Im Falle, daß R⁷ und R⁸ jeweils ein Metallkation oder quarternäres Ammoniumkation bedeuten, können die Verbindungen II und III auch ohne Hilfsbase im Molverhältnis von etwa 1:1 eingesetzt werden.

Sulfurylchlorid (Verbindung der Formel IV) wird vorzugsweise äquimolar zur Verbindung der Formel II oder im Überschuß eingesetzt, beispielsweise im Molverhältnis II:IV von 1:1 bis 1:2, vorzugsweise 1:1 bis 1:1,5. Größere Überschüsse sind ebenfalls möglich. Es ist in der Regel zweckmäßig, im Überschuß zugesetztes Sulfurylchlorid vor der Zugabe der Verbindungen der Formel V destillativ zu entfernen.

Die Verbindungen der Formel V können äquimolar, im Unterschuß oder im Überschuß zu Verbindungen der Formel II eingesetzt werden. Nicht umgesetzte Anteile von V lassen sich nach üblichen Methoden aus dem Reaktionsgemisch abtrennen und wieder für das Verfahren einsetzen.

Die für die Herstellung der Verbindungen der allgemeinen Formel I nach dem erfindungsgemäßen Verfahren benötigten Ausgangsverbindungen der Formel III und IV sind im Handel erhältlich oder nach allgemein bekannten Verfahren leicht zugänglich. Die Verbindungen der Formel II sind entweder im Handel erhältlich oder analog üblicher Methoden, z. B. mittels Umsetzung von Sulfochloriden mit Aminen, herstellbar.

Die Heterocyclen der Formel V sind ebenfalls entweder im Handel erhältlich oder lassen sich nach geeigneten Methoden leicht darstellen; siehe z. B. US-A-4,310,470, EP-A-0027200, US-A-4,299,960, M. J. Langermann, C. K. Banks, J. Am. Chem. Soc. 73, 3011 (1951).

Ein Vorteil des erfindungsgemäßen Verfahrens besteht darin, daß nicht umgesetzte Anteile der Verbindungen der Formeln IV und V sowie die verwendeten Lösungsmittel praktisch quantitativ zurückgewonnen und wieder im Verfahren eingesetzt werden können.

Auch schwerlösliche Nebenkomponenten, wie beispielsweise Natriumchlorid, können zwischen den Reaktionsstufen abgetrennt werden.

Ein zusätzlicher Vorteil des erfindungsgemäßen Verfahrens besteht in der Regel darin, daß die gewünschten Produkte der Formel I, gegebenenfalls nach Zusatz von Wasser oder anderen polaren Lösungsmitteln, als schwerlösliche Verbindungen aus dem Reaktionsmedium in hoher Reinheit ausfallen.

In den folgenden Beispielen beziehen sich Prozentangaben auf das Gewicht, sofern nichts anderes angegeben ist.

### Beispiele

1. 1-[(N-Methylsulfonyl-N-methyl-amino)-sulfonyl]-3-(4,6-dimethoxy-2-pyrimidyl)-harnstoff
   340 g (2,52 mol) Sulfurylchlorid werden in 1,5 l Acetonitril vorgelegt und portionsweise innerhalb von 20 min unter starkem Rühren mit 260 g (4,0 mol) Natriumcyanat versetzt (20 °C). Man läßt 15 min nachrühren, tropft dann 222 g (2,0 mol) Methansulfonsäure-N-methylamid bei 25 °C zu, steigert die Temperatur langsam und erwärmt 200 min unter kräftigem Rühren zum Rückfluß. Anschließend wird bei 100 mbar das überschüssige Sulfurylchlorid mit dem Lösungsmittel bis zu einer Innentemperatur von 50 °C abdestilliert. Nach Entspannen unter Stickstoff werden 1,5 l Acetonitril (wahlweise auch Toluol etc.) zugesetzt und bei 0 °C 155 g (1,0 mol) 2-Amino-4,6-dimethoxy-pyrimidin eingetragen. Nach 75 min wird mit 1,0 l Wasser versetzt, der Niederschlag abgesaugt und gewaschen. Man erhält 304 g 1-[(N-Methylsulfonyl-N-methylamino)-sulfonyl]-3-(4,6-dimethoxy-2-pyrimidyl)-harnstoff vom Schmelzpunkt 176 bis 178 °C. Das Produkt stimmt mit einer Vergleichsprobe überein und weist nach Analyse mittels Hochdruckfüssigkeit-Chromatographie (HPLC) eine Reinheit von 96 % auf; Ausbeute: 77 % d. Th.
2. 1-(2-Ethoxyphenoxysulfonyl)-3-(4,6-dimethoxy-2-pyrimidyl)-harnstoff
   26,0 g (0,4 mol) zerkleinertes Natriumcyanat werden bei Raumtemperatur in 200 ml Acetonitril suspendiert und mit 28,3 g (0,21 mol) Sulfurylchlorid innerhalb von 20 min versetzt, worauf die Temperatur auf 44 °C ansteigt. Nach vierstündigem Nachrühren bei 50 °C wird unter reduziertem Druck andestilliert, auf 27 °C abgekühlt und innerhalb von 10 min mit 27,6 g (0,2 mol) 2-Ethoxyphenol versetzt. Man läßt über Nacht stehen und gibt bei Raumtemperatur 15,5 g (0,1 mol) 2-Amino-4,6-dimethoxypyrimidin zu. Es wird 120 min bei 50 °C nachgerührt, das Lösungsmittel unter reduziertem Druck entfernt, mit 100 ml Wasser versetzt und mit Dichlormethan extrahiert. Nach Abdestillieren des organischen Lösungsmittels bleiben 49,9 g Feststoff zurück, der nach HPLC einen Gehalt von 71,6 Gew-% 1-(2-Ethoxyphenoxysulfonyl)-3-(4,6-dimethoxy-2-pyrimidyl)-harnstoff aufweist. Die Ausbeute beträgt damit ca. 89 % d. Th..

Analog den Beispielen 1 und 2 werden auch die in der folgenden Tabelle aufgeführten Verbindungen der Formel I (Y = CH) erhalten.

| Beispiel | R¹ | X | R³ | R⁴ | R⁵ | R⁶ | Fp.[°C] |
|---|---|---|---|---|---|---|---|
| 3 | CH₃ | SO₂N(C₃H₇) | CH₃ | CH₃ | H | H | 154-157 |
| 4 | CH₃ | SO₂N[CH(CH₃)₂] | CH₃ | CH₃ | H | H | 120-122 |
| 5 | C₂H₅ | SO₂N(C₂H₅) | CH₃ | CH₃ | H | H | |
| 6 | CH₃ | SO₂N(CH₃) | CH₃ | CH₃ | H | H | |
| 7 | CH₃ | SO₂N(CH₃) | CH₃ | CH₃ | H | CH₃ | |
| 8 | C₄H₉ | SO₂N(CH₃) | CH₃ | CH₃ | H | H | |
| 9 | CH₃ | SO₂N(Cyclohexyl) | CH₃ | CH₃ | H | CH₃ | |
| 10 | 2-i-PrO-C₆H₄ | O | OCH₃ | CH₃ | H | H | 141-143 |
| i-PrO = Isopropoxy | | | | | | | |

## Patentansprüche

1. Verfahren zur Herstellung von Verbindungen der Formel I oder deren Salzen, 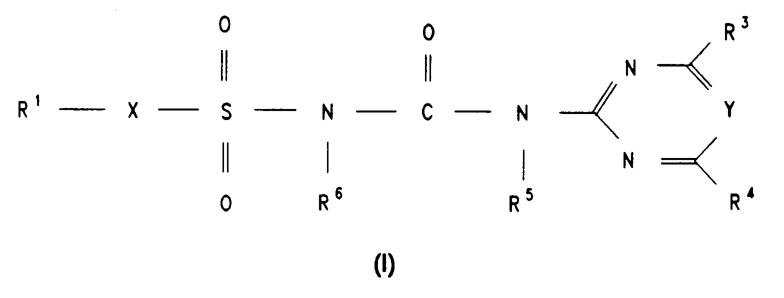 worin
X Sauerstoff, -O-NR²-, -SO₂-NR²-,
Y Stickstoff oder CH,
R¹ (C₁-C₆)Alkyl, (C₂-C₆)Alkenyl oder (C₂-C₆)Alkinyl, wobei jeder der letztgenannten drei Reste unabhängig voneinander unsubstituiert oder durch einen oder mehrere Reste aus der Gruppe Halogen, (C₁-C₄)Alkoxy und [(C₁-C₄)Alkoxy]-carbonyl substituiert ist,
oder, im Falle von X = Sauerstoff, auch Phenyl, das unsubstituiert oder durch einen oder mehrere Reste aus der Gruppe Halogen, Nitro, (C₁-C₄)Alkyl, (C₁-C₄)Haloalkyl, (C₁-C₄)Alkoxy, (C₁-C₄)Haloalkoxy und [(C₁-C₄)Alkoxy]-carbonyl substituiert ist,
R² Wasserstoff, (C₁-C₆)Alkyl, (C₂-C₆)Alkenyl, (C₂-C₆)Alkinyl oder (C₃-C₆)Cycloalkyl,
R³, R⁴ unabhängig voneinander Wasserstoff, (C₁-C₄)Alkyl oder (C₁-C₄)Alkoxy, wobei jeder der letztgenannten beiden Reste unsubstituiert oder durch einen oder mehrere Reste aus der Gruppe Halogen, Alkoxy und Alkylthio substituiert ist, oder Halogen, (C₁-C₄)Alkylthio, (C₁-C₄)Alkylamino oder Di[(C₁-C₄)alkyl]-amino,
R⁵ Wasserstoff oder (C₁-C₄)Alkyl und
R⁶ Wasserstoff bedeuten,
dadurch gekennzeichnet, daß man Verbindungen der Formel II,
R¹ - X - R⁷ (II)
worin R¹ und X wie in Formel I definiert sind und R⁷ Wasserstoff, ein quarternäres Ammoniumion oder ein Äquivalent eines ein-, zwei- oder mehrwertigen Metallkations ist, mit Verbindungen der Formeln III, IV und V,
R⁸ - OCN (III)
SO₂Cl₂ (IV) 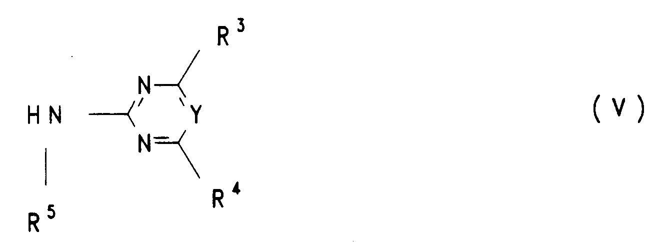
in denen R³, R⁴, R⁵, X und Y wie in Formel I definiert sind und R⁸ Wasserstoff, ein quarternäres Ammoniumion oder ein Äquivalent eines ein-, zwei- oder mehrwertigen Metallkations bedeutet, umsetzt.

2. Verfahren nach Anspruch 1, dadurch gekennzeichnet, daß
R¹X N-[(C₁-C₆)Alkylsulfonyl]-N-[(C₁-C₃)-alkyl]-amino oder [(C₁-C₄)Alkoxy]-phenoxy,
R³, R⁴ unabhängig voneinander (C₁-C₂)Alkyl oder (C₁-C₂)Alkoxy,
R⁵ Wasserstoff oder Methyl und
R⁶ Wasserstoff bedeuten.

3. Verfahren nach Anspruch 1 oder 2, dadurch gekennzeichnet, daß R¹X N-[(C₁-C₃)-Alkylsulfonyl]-N-[(C₁-C₂)-alkyl]-amino oder (C₁-C₃)-Alkoxyphenoxy bedeutet.

4. Verfahren nach einem der Ansprüche 1 bis 3, dadurch gekennzeichnet, daß R³ und R⁴ Methyl oder Methoxy bedeuten.

5. Verfahren nach einem der Ansprüche 1 bis 4, dadurch gekennzeichnet, daß man zunächst die Verbindungen der Formeln II, III und IV miteinander umsetzt und danach die Umsetzung mit Verbindungen der Formel V durchführt.

6. Verfahren nach Anspruch 5, dadurch gekennzeichnet, daß die Reaktionstemperatur für die Umsetzungen der Verbindungen der Formeln II, III und IV zwischen 0 °C und 200 °C ist.

7. Verfahren nach Anspruch 5 oder 6, dadurch gekennzeichnet, daß die Reaktionstemperatur für die Umsetzung mit der Verbindung der Formel V zwischen -20 °C und 120 °C liegt.

8. Verfahren nach einem der Ansprüche 1 bis 7, dadurch gekennzeichnet, daß das Verfahren oder einzelne Verfahrensschritte in Gegenwart von unter den Reaktionsbedingungen inerten anorganischen oder organischen Lösungsmitteln oder in Gemischen dieser Lösungsmittel durchgeführt wird.

9. Verfahren nach Anspruch 8, dadurch gekennzeichnet, daß ein Lösungsmittel aus der Gruppe aprotische polare organische Lösungsmittel, aliphatische und aromatische, gegebenenfalls halogenierte Kohlenwasserstoffe und Gemische der genannten organischen Lösungsmittel eingesetzt wird.

10. Verfahren nach einem der Ansprüche 1 bis 9, dadurch gekennzeichnet, daß die Verbindungen der Formeln II, III und IV unter aprotischen Bedingungen umgesetzt werden.

11. Verfahren nach Anspruch 10, dadurch gekennzeichnet, daß
R⁸ ein Metallkation oder quarternäres Ammoniumkation ist
und die Verbindungen der Formeln II und III
a) im Falle R⁷ = H, im Molverhältnis von höchstens 1:2 oder
b) im Falle R⁷ = H, im Molverhältnis von etwa 1:1 und in Gegenwart von 1 Moläquivalent einer Hilfsbase, die von Verbindung III verschieden ist, oder
c) im Falle R⁷ = Metallkation oder quarternäres Ammoniumkation, im Molverhältnis von etwa 1:1 ohne Hilfsbase
eingesetzt werden.

12. Verfahren nach einem der Ansprüche 1 bis 11, dadurch gekennzeichnet, daß die Verbindung der Formel IV äquimolar zur Verbindung der Formel II oder im Überschuß eingesetzt wird.

13. Verfahren zur Herstellung einer Additionsverbindung ("Addukt 1"), dadurch gekennzeichnet, daß man eine Verbindung der Formel III,
R⁸ - OCN (III)
worin
R⁸ Wasserstoff, ein quarternäres Ammoniumion oder ein Äquivalent eines ein-, zwei- oder mehrwertigen Metallkations bedeutet,
mit einer Verbindung der Formel IV,
SO₂Cl₂ (IV)
umsetzt.

14. Verfahren nach Anspruch 13, dadurch gekennzeichnet, daß man die Umsetzung bei einer Reaktionstemperatur von 0 bis 80°C ohne Lösungsmittel oder in Gegenwart eines aprotischen polaren organischen oder aprotischen polaren anorganischen Lösungsmittels durchführt.

15. Additionsverbindung ("Addukt 1") erhältlich nach dem Verfahren nach Anspruch 13 oder 14.

16. Verfahren zur Herstellung einer Additionsverbindung ("Addukt 2"), dadurch gekennzeichnet, daß man eine Additionsverbindung ("Addukt 1") nach Anspruch 15 mit einer Verbindung der Formel II,
R¹ - X - R⁷ (II)
worin R¹ und X wie in Formel I nach einem der Ansprüche 1 bis 4 definiert sind und R⁷ Wasserstoff, ein quarternäres Ammoniumion oder ein Äquivalent eines ein-, zwei- oder mehrwertigen Metallkations ist, umsetzt.

17. Verfahren nach Anspruch 16, dadurch gekennzeichnet, daß die Umsetzung bei einer Reaktionstemperatur von 0 bis 200°C in Gegenwart eines aprotischen polaren organischen oder aprotischen polaren anorganischen Lösungsmittel durchgeführt wird.

## Claims

1. A process for preparing compounds of the formula I or salts thereof, 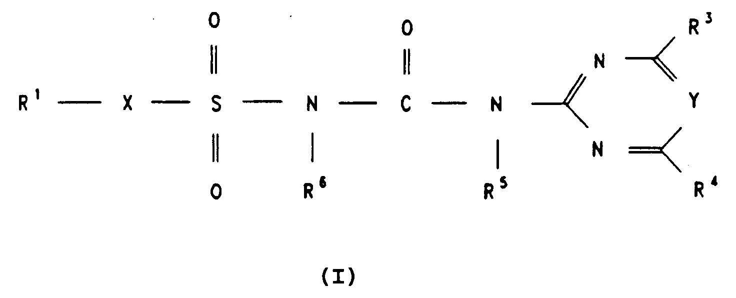 in which
X is oxygen, -O-NR²- or -SO₂-NR²-,
Y is nitrogen or CH,
R¹ is (C₁-C₆)alkyl, (C₂-C₆)alkenyl or (C₂-C₆)-alkynyl, where each of the latter three radicals, independently of each other, is unsubstituted or substituted by one or more radicals selected from the group comprising halogen, (C₁-C₄)alkoxy and [(C₁-C₄)alkoxy]-carbonyl,
or, in the case where X = oxygen, also phenyl, which is unsubstituted or substituted by one or more radicals selected from the group comprising halogen, nitro, (C₁-C₄)alkyl, (C₁-C₄)haloalkyl, (C₁-C₄)-alkoxy, (C₁-C₄)haloalkoxy and [(C₁-C₄)-alkoxy]-carbonyl,
R² is hydrogen, (C₁-C₆)alkyl, (C₂-C₆)alkenyl, (C₂-C₆)alkynyl or (C₃-C₆)cycloalkyl,
R³ and R⁴, independently of each other, are hydrogen, (C₁-C₄)alkyl or (C₁-C₄)alkoxy, where each of the latter two radicals is unsubstituted or substituted by one or more radicals selected from the group comprising halogen, alkoxy and alkylthio, or halogen, (C₁-C₄)alkylthio, (C₁-C₄)-alkylamino or di[(C₁-C₄)alkyl]-amino,
R⁵ is hydrogen or (C₁-C₄)alkyl and
R⁶ is hydrogen,
which comprises reacting compounds of the formula II,
R¹ - X - R⁷ (II)
in which R¹ and X are defined as in formula I and R⁷ is hydrogen, a quaternary ammonium ion or one equivalent of a singly, doubly or multiply charged metal cation,
with compounds of the formulae III, IV and V,
R⁸ - OCN (III)
SO₂Cl₂ (IV) 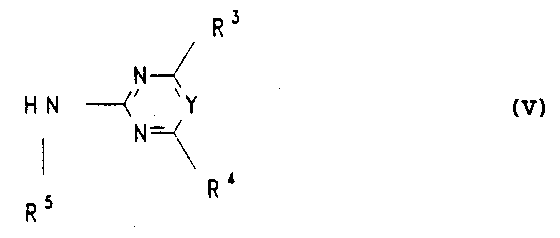
in which R³, R⁴, R⁵, X and Y are defined as in formula I and R⁸ is hydrogen, a quaternary ammonium ion or one equivalent of a singly, doubly or multiply charged metal cation.

2. The process as claimed in claim 1, wherein
R¹X is N-[(C₁-C₆)-alkylsulfonyl]-N-[(C₁-C₃)-alkyl]-amino or [(C₁-C₄)alkoxy]-phenoxy,
R³ and R⁴, independently of each other, are (C₁-C₂)alkyl or (C₁-C₂)alkoxy,
R⁵ is hydrogen or methyl and
R⁶ is hydrogen.

3. The process as claimed in claim 1 or 2, wherein R¹X is N-[(C₁-C₃)-alkylsulfonyl]-N-[(C₁-C₂)-alkyl]-amino or (C₁-C₃)-alkoxyphenoxy.

4. The process as claimed in one of claims 1 to 3, wherein R³ and R⁴ are methyl or methoxy.

5. The process as claimed in one of claims 1 to 4, wherein the compounds of the formulae II, III and IV are first reacted with each other and the reaction with compounds of the formula V is carried out thereafter.

6. The process as claimed in claim 5, wherein the reaction temperature for the reactions of the compounds of the formulae II, III and IV is between 0°C and 200°C.

7. The process as claimed in claim 5 or 6, wherein the reaction temperature for the reaction with the compound of the formula V is between -20°C and 120°C.

8. The process as claimed in one of claims 1 to 7, wherein the process or individual stages of the process are carried out in the presence of inorganic or organic solvents which are inert under the reaction conditions, or in mixtures of these solvents.

9. The process as claimed in claim 8, wherein a solvent is employed selected from the group comprising aprotic polar organic solvents, aliphatic and aromatic, where appropriate halogenated, hydrocarbons, and mixtures of the said organic solvents.

10. The process as claimed in one of claims 1 to 9, wherein the compounds of the formulae II, III and IV are reacted under aprotic conditions.

11. The process as claimed in claim 10, wherein
R⁸ is a metal cation or a quaternary ammonium cation
and the compounds of the formulae II and III are employed
a) in the case where R⁷ = H, in a molar ratio of at most 1:2 or
b) in the case where R⁷ = H, in a molar ratio of about 1:1 and in the presence of 1 mole equivalent of an auxiliary base which is different from compound III or
c) in the case where R⁷ = metal cation or quaternary ammonium cation, in a molar ratio of about 1:1 without auxiliary base.

12. The process as claimed in one of claims 1 to 11, wherein the compound of the formula IV is equimolar to the compound of the formula II, or is employed in excess.

13. A process for preparing an addition compound ("adduct 1"), which comprises reacting a compound of the formula III,
R⁸ - OCN (III)
in which
R⁸ is hydrogen, a quaternary ammonium ion or one equivalent of a singly, doubly or multiply charged metal cation,
with a compound of the formula IV,
SO₂Cl₂ (IV).

14. The process as claimed in claim 13, wherein the reaction is carried out at a reaction temperature of 0 to 80°C without solvent or in the presence of an aprotic polar organic or aprotic polar inorganic solvent.

15. An addition compound ("adduct 1") obtainable by the process as claimed in claim 13 or 14.

16. A process for preparing an addition compound ("adduct 2"), which comprises reacting an addition compound ("adduct 1") as claimed in claim 15 with a compound of the formula II,
R¹ - X - R⁷ (II)
in which R¹ and X are defined as in formula I as claimed in one of claims 1 to 4 and R⁷ is hydrogen, a quaternary ammonium ion or one equivalent of a singly, doubly or multiply charged metal cation.

17. The process as claimed in claim 16, wherein the reaction is carried out at a reaction temperature of 0 to 200°C in the presence of an aprotic polar organic or aprotic polar inorganic solvent.

## Revendications

1. Procédé pour la préparation de composés de formule I ou de leurs sels 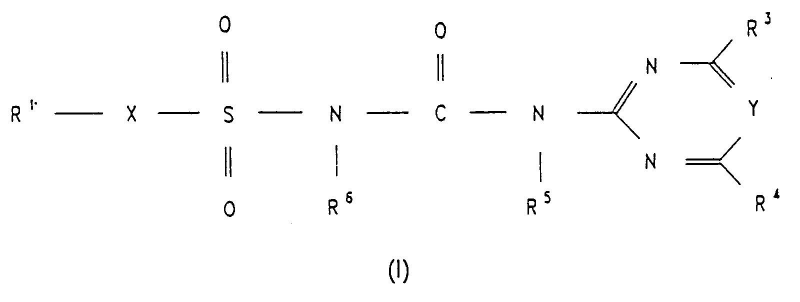 où
X est l'oxygène, -O-NR²- ou -SO₂-NR²-,
Y est l'azote ou CH,
R¹ représente alkyle en C₁-C₆, alcényle en C₂-C₆ ou alcynyle en C₂-C₆, chacun de ces trois radicaux, indépendamment l'un de l'autre, étant non substitué ou substitué par un ou plusieurs radicaux pris dans le groupe comportant les halogènes, alcoxyle en C₁-C₄ et (alcoxyle en C₁-C₄)-carbonyle,
ou, dans le cas où X est l'oxygène, est également phényle qui est non substitué ou substitué par un ou plusieurs radicaux pris dans le groupe comportant les halogènes, nitro, alkyle en C₁-C₄, haloalkyle en C₁-C₄, alcoxy en C₁-C₄, haloalcoxy en C₁-C₄ et (alcoxy en C₁-C₄)carbonyle,
R² est l'hydrogène, alkyle en C₁-C₆, alcényle en C₂-C₆, alcynyle en C₂-C₆ ou cycloalkyle en C₃-C₆,
R³, R⁴ indépendamment l'un de l'autre sont l'hydrogène, alkyle en C₁-C₄ ou alcoxy en C₁-C₄, les deux derniers radicaux étant non substitués ou substitués par un ou plusieurs radicaux pris dans le groupe comportant, les halogènes, alcoxy et alkylthio, ou les halogènes, (alkyle en C₁-C₄)-thio, (alkyle en C₁-C₄)-amino ou di(alkyle en C₁-C₄)-amino et
R⁵ est l'hydrogène ou alkyle en C₁-C₄ et
R⁶ l'hydrogène,
caractérisé en ce qu'on fait réagir des composés de formule II
R¹ - X - R⁷ (II)
où R¹ et X sont définis dans la formule I et R⁷ est l'hydrogène, un ion ammonium quaternaire ou un équivalent d'un cation de métal mono-, di- ou multivalent, avec des composés de formules III, IV et V
R⁸ - OCN (III)
SO₂Cl₂ (IV) (IV) 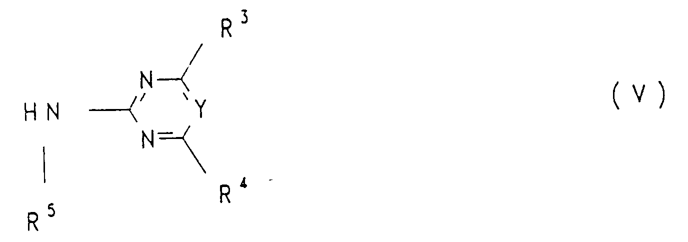
dans lesquelles R³, R⁴, R⁵, X et Y sont définis comme dans la formule I et R⁸ est l'hydrogène, un ion ammonium quaternaire ou un équivalent d'un cation métallique mono-, di- ou multivalent.

2. Procédé selon la revendication 1 caractérisé en ce que
R¹X représente N[(alkyl en C₁-C₆)-sulfonyl]-N-[alkylène C₁-C₂]-amino ou [alcoxy en C₁-C₄]-phénoxy
R³, R⁴, indépendamment l'un de l'autre, sont alkyle en C₁-C₂ ou alcoxy en C₁-C₂,
R⁵ est l'hydrogène ou méthyle, et
R⁶ représente l'hydrogène.

3. Procédé selon la revendication 1 ou 2, caractérisé en ce que R¹X est N-[(alkylène C₁-C₃)-sulfonyl]-N-[alkyl en C₁-C₂]-amino ou (alkoxy en C₁-C₃)-phénoxy.

4. Procédé selon l'une des revendications 1 à 3, caractérisé en ce que R³ et R⁴ sont méthyle ou méthoxy.

5. Procédé selon l'une des revendications 1 à 4, caractérisé en ce qu'on fait réagir d'abord les composés de formules II, III et IV ensemble, et ensuite on met en oeuvre la réaction des composés de formule V.

6. Procédé selon la revendication 5, caractérisé en ce que la température de réaction pour la conversion des composés de formules II, III et IV est comprise entre 0°C et 200°C.

7. Procédé selon la revendication 5 ou 6, caractérisé en ce que la température de réaction pour la conversion des composés de formule V est comprise entre -20°C et 120°C.

8. Procédé selon l'une des revendications 1 à 7, caractérisé en ce qu'on met en oeuvre le procédé ou les étapes individuelles du procédé en présence de solvants inorganiques ou organiques inertes dans des conditions de réaction ou dans les mélanges de ces solvants.

9. Procédé selon la revendication 8, caractérisé en ce qu'on utilise un solvant pris dans le groupe des solvants organiques polaires aprotiques, aliphatiques et aromatiques, éventuellement des hydrocarbures halogénés et des mélanges des solvants organiques précités.

10. Procédé selon l'une des revendications 1 à 9, caractérisé en ce qu'on fait réagir les composés de formule II, III et IV dans des conditions aprotiques.

11. Procédé selon la revendication 10 caractérisé en ce que
R⁸ est un cation de métal ou un cation ammonium quaternaire
et les composés de formules II et III sont utilisés
a) dans le cas où R⁷ est l'hydrogène, dans un rapport en moles tout au plus de 1:2, ou
b) dans le cas où R⁷ est l'hydrogène, dans un rapport en moles d'environ 1:1 et en présence d'un équivalent en moles d'une base auxiliaire, qui est différente du composé III, ou
c) dans le cas où R⁷ est un cation de métal ou un cation ammonium quaternaire, dans un rapport en moles d'environ 1:1 sans base auxiliaire.

12. Procédé selon l'une des revendications 1 à 11, caractérisé en ce qu'on utilise le composé de formule IV en quantité équimolaire par rapport au composé de formule II, ou en excès.

13. Procédé pour la préparation d'un composé d'addition (produit d'addition 1) caractérisé en ce qu'on fait réagir un composé de formule III
R⁸ - OCN (III)
où
R⁸ est l'hydrogène, un ion ammonium quaternaire ou un équivalent d'un cation de métal mono-, di- ou multivalent,
avec un composé de formule IV
SO₂Cl₂ (IV).

14. Procédé selon la revendication 13 caractérisé en ce qu'on met en oeuvre la réaction à une température de réaction de 0 à 80°C, sans solvant ou en présence d'un solvant organique polaire aprotique ou d'un solvant inorganique polaire aprotique.

15. Composé d'addition ("produit d'addition 1") que l'on peut obtenir selon le procédé selon les revendications 13 ou 14.

16. Procédé pour la préparation d'un composé d'addition ("produit d'addition 2") caractérisé en ce qu'on fait réagir un composé d'addition ("produit d'addition 1") selon la revendication 15 avec un composé de formule II
R¹ - X - R⁷ (II)
dans laquelle R¹ et X sont définis comme dans la formule I selon l'une des revendications 1 à 4, et R⁷ représente l'hydrogène, un ion ammonium quaternaire ou un équivalent d'un cation de métal mono, di ou multivalent.

17. Procédé selon la revendication 16, caractérisé en ce qu'on met en oeuvre la réaction à une température de réaction de 0 à 200°C, en présence d'un solvant organique polaire aprotique ou d'un solvant inorganique polaire aprotique.
